# EUROPÄISCHE PATENTSCHRIFT

(11) **EP 3 780 990 B1**
(45) Veröffentlichungstag und Bekanntmachung des Hinweises auf die Patenterteilung: **15.06.2022**
(21) Anmeldenummer: 19722807.5
(22) Anmeldetag: 18.04.2019
(51) Int. Cl.: A45C 11/00, A61L 12/08

(54) **KONTAKTLINSEN-AUFBEWAHRUNGS- UND REINIGUNGSBEHÄLTER**
CONTACT LENS STORAGE AND CLEANING CONTAINER
RÉSERVOIR DE STOCKAGE ET DE NETTOYAGE DE LENTILLES DE CONTACT

(30) Priorität: 19.04.2018 EP 18168315
(43) Veröffentlichungstag der Anmeldung: 24.02.2021
(73) Patentinhaber: Doniga, Ioan Cornelius, 55116 Mainz (DE)
(72) Erfinder: Doniga, Ioan Cornelius, 55116 Mainz (DE)
(74) Vertreter: Tergau & Walkenhorst Patentanwälte PartGmbB
(86) Internationale Anmeldenummer: PCT/EP2019/060105
(87) Internationale Veröffentlichungsnummer: WO 2019/202084

(56) Entgegenhaltungen:
- EP-B1- 2 095 735
- WO-A2-2015/150262
- DE-U1-202008 002 795
- DE-U1-202008 002 795
- FR-A1- 2 835 751
- US-A1- 2011 284 772

## Beschreibung

Die Erfindung betrifft einen Kontaktlinsen-Aufbewahrungs- und -Reinigungsbehälter mit einer Anzahl von verschließbaren Kontaktlinsenkammern, denen mittels einer Pumpvorrichtung über ein Kanalsystem Kontaktlinsenflüssigkeit aus einem Flüssigkeitsreservoir zuführbar ist.

Kontaktlinsen sind ein weltweit verbreitetes Sehhilfsmittel. Sie ersetzen die meist nicht so ästhetische Brille, indem sie der Augenhornhaut direkt anliegen, nur durch einen dünnen Tränenfilm von dieser getrennt. Es gibt verschiedene Kontaktlinsentypen, insbesondere Tages-, Monats- und Jahreskontaktlinsen. Tageskontaktlinsen sollten nur einen Tag lang getragen und dann entsorgt werden, wohingegen Monatskontaktlinsen 30 Tage und Jahreskontaktlinsen bis zu einem Jahr lang genutzt werden können.

Üblicherweise müssen Kontaktlinsen, gerade bei regelmäßigem Gebrauch, regelmäßig gepflegt und sauber gehalten werden. Dazu werden Kontaktlinsen üblicherweise zur vorübergehenden Aufbewahrung, beispielsweise zu Nachtzeiten, und zur Pflege in zugeordnete Kontaktlinsenbehälter gelegt und dort in eine Aufbewahrungs- und Reinigungsflüssigkeit eingelegt. Die Einbringung der Linse in die Aufbewahrungs- und Reinigungsflüssigkeit dient einerseits zur Desinfektion und Reinigung der Kontaktlinse, soll andererseits aber auch ein Austrocknen der Kontaktlinse verhindern.

Eine solche Behandlung der Kontaktlinsen ist für den Nutzer vergleichsweise aufwendig, insbesondere da üblicherweise sowohl ein Aufbewahrungs- und Reinigungsbehälter für die Kontaktlinsen als auch ein Flüssigkeitsspender für die Pflegeflüssigkeit bereitgehalten werden muss. Außerdem ist das ständige Umfüllen von Pflegeflüssigkeit vom Flüssigkeitsspender in die Kontaktlinsenkammern hinein aufgrund der geringen Größe dieser Kontaktlinsenkammern mit einem gewissen unbeabsichtigten Verlust von Pflegeflüssigkeit verbunden und auf die Dauer lästig. Um diesen Missständen abzuhelfen, ist aus der EP 2095735 B1 ein Kontaktlinsen-Aufbewahrungs- und -Reinigungsbehälter der oben genannten Art bekannt, bei dem in der Art einer integrierten Ausführung die zum vorübergehenden Verwahren der Kontaktlinsen vorgesehenen Kontaktlinsenkammern und das Reservoir zum Vorhalten der Pflegeflüssigkeit im Umfang des Bedarfs von mindestens einigen Tagen in einem einzigen, gemeinsamen Körper oder Gehäuse angeordnet sind. Durch diese Ausgestaltung ist eine sehr einfach zu handhabende und auch sichere Verwahrung der Kontaktlinsen ermöglicht, wobei zudem auch durch eine strikte Medientrennung zuverlässig eine Vermischung der frischen, unverbrauchten Flüssigkeit mit Luft oder mit anderen, potentiell mit Keimen behafteten Medien vermieden werden kann. Zur Einbringung der Pflegeflüssigkeit in die Kontaktlinsenkammern sind diese über ein Kanalsystem mit dem Reservoir für die Kontaktlinsenflüssigkeit verbunden. Die Kontaktlinsenflüssigkeit kann dabei mittels einer Pumpvorrichtung aus dem Reservoir in die Kontaktlinsenkammern eingespeist werden. Ein weiterer Kontaktlinsen-Aufbewahrungs- und -Reinigungsbehälter ist aus FR 2,835,751 A1 bekannt.

Der Erfindung liegt die Aufgabe zugrunde, einen Kontaktlinsen-Aufbewahrungs- und -Reinigungsbehälter der beschriebenen Art anzugeben, mit dem ohne den Bedarf einer Nachbefüllung die Bereitstellung der Flüssigkeit zur Pflege der Kontaktlinsen für einen besonders langen Zeitraum von beispielsweise einigen Wochen ermöglicht ist.

Diese Aufgabe wird erfindungsgemäß durch einen Kontaktlinsen-Aufbewahrungs- und -Reiningungsbehälter gemäß Anspruch 1 gelöst.

Vorteilhafte Ausgestaltungen der Erfindung sind Gegenstand der Unteransprüche. Die Erfindung geht dabei von der Überlegung aus, dass eine wesentliche Ursache für eine zeitliche Begrenzung der Bevorratung und Bereitstellung der Pflegeflüssigkeit in einem Behälter der genannten Art in einem Risiko der Verkeimung der vorgehaltenen Flüssigkeit gesehen werden sollte. Um diesem Risiko entgegenzuwirken und damit die Bevorratung für einen besonders langen Zeitraum zu ermöglichen, sollte die Möglichkeit eines Keimeintrags in das Flüssigkeitsreservoir konsequent minimiert werden. In einem Behälter nach der Bauweise der genannten Art ist das Flüssigkeitsreservoir in den Innenkörper oder die Kartusche des Behälters integriert, so dass ein Kontakt mit Umgebungsluft oder anderen Keimquellen bereits weitgehend vermieden ist. Allerdings wird über den Flüssigkeitskanal, über den das Reservoir mit den Kontaktlinsenkammern verbunden ist, möglicherweise und gelegentlich eine Verbindung mit der Außenumgebung hergestellt.

Um einen potentiellen Keimeintrag auch über diese Verbindungslinie konsequent auszuschließen, sollte ein Spülgefälle sichergestellt werden, über das der Medienfluss vom Reservoir weg zum Außenbereich aufgebaut wird. Dies wird erreicht, indem über das Kanalsystem eine kaskadenartige Hintereinanderschaltung von Reservoir, Kontaktlinsenkammern und einer Auslass- oder Entsorgungsöffnung für verbrauchte Pflegeflüssigkeit einstellbar ist. Zur Entsorgung von in den Linsenkammern befindlicher verbrauchter Flüssigkeit kann dann über diese Einstellung eine Neubefüllung der Linsenkammern mit unverbrauchter Flüssigkeit aus dem Reservoir erfolgen, wobei gleichzeitig die alte, verbrauchte Flüssigkeit von den Linsenkammern aus über die Auslassöffnung ausgeschoben wird. Eine Rückströmung von außen in das Reservoir hinein, die zu einem unerwünschten Keimeintrag führen könnte, ist damit weitgehend ausgeschlossen.

Die Auslassöffnung kann dabei in der Art eines "echten" Auslasses an einer Außenoberfläche oder -seite des Behälters angeordnet sein, so dass die verbrauchte Flüssigkeit über die Auslassöffnung ausgeschoben und einer Entsorgung direkt zugeführt werden kann. Alternativ kann die Auslassöffnung aber auch im Inneren des Behälters angeordnet sein und in ein separates, integriertes Auffangreservoir für die verbrauchte Flüssigkeit münden. In dieser Version, in der kein externer Auslass mehr vorgesehen sein muss, kann die Entsorgung dann beispielsweise über einen Austausch des vollständigen, die verbrauchte Flüssigkeit enthaltenden Auffangreservoirs erfolgen.

In einer besonders bevorzugten Ausführungsform kann das Reservoir in der Art eines "Doppelkammerreservoirs", vorzugsweise doppelwandig, ausgeführt sein, wobei eine Reservoirkammer zur Vorhaltung frischer, unverbrauchter Linsenflüssigkeit und die andere Reservoirkammer zur vorübergehenden oder endgültigen Aufnahme der verbrauchten Flüssigkeit vorgesehen ist. Die Auslassöffnung mündet dabei nicht in den Außenbereich des Gesamtsystems, sondern in die Aufnahmekammer, so dass eine Abgabe der verbrauchten Flüssigkeit an die Außenwelt vermieden werden kann. In der bevorzugten doppelwandigen Ausführung ist die für die zwischenzeitliche (oder endgültige) Aufnahme der verbrauchten Flüssigkeit vorgesehene Aufnahmekammer außerhalb der eigentlichen Reservoirkammer und diese umgebend angeordnet.

Gemäß der Erfindung ist als Pumpvorrichtung eine Membranpumpe vorgesehen. Damit ist auf besonders einfache und auch kostengünstige Weise eine zuverlässige Pumpfunktion gewährleistet. In alternativer vorteilhafter nicht Erfindungsgemäßer Ausgestaltung ist als Pumpvorrichtung eine Kolbenpumpe oder eine Faltenbalgpumpe vorgesehen.

Erfindungsgemäß weist der Behälter eine in einem Außengehäuse angeordnete Kartusche auf, in die das Flüssigkeitsreservoir integriert ist. Eine solche Bauweise ermöglicht eine besonders einfache Auffüllung, nachdem die im Reservoir vorgehaltene Flüssigkeit aufgebraucht ist. Dazu kann nämlich die vollständige Kartusche ersetzt werden. Das Risiko einer Verkeimung der Flüssigkeit während des Austauschs ist dabei nahezu ausgeschlossen, da während des Austauschvorgangs zu keiner Zeit eine medienseitige Verbindung zwischen dem Innenraum des Reservoirs und der Außenumgebung hergestellt wird. Insbesondere in Kombination mit dem Spülgefälle durch die kaskadenartige Hintereinanderschaltung von Kontaktlinsenkammern und Auslassöffnung kann nach einem solchen Austausch durch Ausbringung einer kleinen Menge der neu beigebrachten Pflegeflüssigkeit ein vollständiger Spülvorgang vom Inneren nach außen hin vorgenommen werden, so dass eventuell vorhandene Keime ausgespült und vom Flüssigkeitsreservoir ferngehalten werden. Beim Austausch ist somit die vorhandene, "verbrauchte" Kartusche aus dem Gehäuse herausnehmbar und durch eine neue Kartusche ersetzbar; das Füllvolumen innerhalb der Kartusche für die Pflegeflüssigkeit beträgt in einer vorteilhaften Weiterbildung etwa 100 ml.

In einer Ausführungsform der Erfindung kann das Kanalsystem ebenfalls in die Kartusche integriert sein. In alternativer zweckmäßiger Ausgestaltung ist das Kanalsystem aber ebenfalls in einem separaten Gehäuseteil angeordnet, das beispielsweise über eine Anzahl von Steckverbindungen mit der Kartusche verbindbar ausgestaltet sein kann.

In einer ebenfalls als vorteilhaft und eigenständig erfinderisch angesehenen Alternative kann - unter Beibehaltung der sonstigen Gestaltungsmerkmale - anstelle einer solchen, mit einer harten Außenschale versehenen Kartusche auch ein Beutel mit weicher, verformbarer Außenhaut vorgesehen sein. Auch in dieser Ausführung ist eine Auffüllung des Systems mit Reinigungsflüssigkeit auf besonders einfache und sicher keimfrei haltbare Weise durch Ersatz des Beutels ermöglicht. Auch in dieser Ausführung ist der Beutel vorzugsweise innerhalb eines vorzugsweise harten Außengehäuses angeordnet und durch dieses geschützt.

In zusätzlicher vorteilhafter Weiterbildung ist die oder jede Kontaktlinsenkammer jeweils durch einen mit einem zugeordneten Kammerdeckel verschließbaren Kammerboden gebildet, wobei der Kammerdeckel insbesondere in der Art einer Deckelmembran flexibel ausgestaltet sein kann, so dass er durch Druck seine Form ändert. Der Kammerboden kann durch eine Vertiefung in der Oberfläche der Kartusche gebildet sein, wobei der jeweilige Kammerdeckel am Außengehäuse angeordnet ist. Durch das Öffnen des Außengehäuses werden somit für sämtliche Kontaktlinsenkammern die Kammerdeckel angehoben und damit geöffnet, so dass sämtliche Kammern gleichermaßen zugänglich gemacht werden. Alternativ können die einzelnen Kammerdeckel aber auch separat und unabhängig voneinander ausgelegt sein, so dass sie unabhängig voneinander geöffnet werden können.

Der Kammerdeckel kann sich allerdings bspw. auch zwischen Kartusche und "System" befinden, wobei das System vorteilhafterweise zumindest eine Pumpmodalität (=Möglichkeit zur Betätigung der Pumpe, nicht unbedingt die Pumpe selbst) beinhaltet. Die eigentliche Pumpe kann dabei beispielsweise durch ein inneres Langloch oder durch seitliche Vorrichtungen betätigt werden.

Vorteilhafterweise ist der oder jeder Kammerdeckel als verformbare Membran ausgeführt. Durch diese Ausführung ist auf besonders einfache Weise die zuverlässige Abdichtung der Linsenkammern im geschlossenen Zustand ermöglicht. Insbesondere kann die Membran derart ausgeführt sein, dass sie an ihrem umlaufenden, den Kontakt mit der Kartuschenoberfläche herstellenden Rand eine Dichtlippe ausbildet. Sobald dann während einer Befüllung mit Flüssigkeit Druck in der Linsenkammer entsteht, wird die Membran nach oben zum Außengehäuse hin verschoben, und sie schafft Platz für die einströmende Flüssigkeit. Gleichzeitig wird dabei Druck auf die seitliche Dichtlippe ausgeübt, so dass sich der Kontakt mit dem "Gegenstück", also der Oberfläche der Kartusche, intensiviert und die Dichtungswirkung verstärkt wird.

In besonders vorteilhafter Weiterbildung kann die Ausgestaltung der Kammerdeckel als verformbare Membran auch zur Bereitstellung einer Indikatorfunktion genutzt werden, die dem Benutzer auf besonders einfache Weise die Feststellung ermöglicht, ob die jeweilige Linsenkammer gerade mit Pflegeflüssigkeit befüllt ist. Dazu wirkt die verformbare Membran vorzugsweise mit einem von außerhalb des Außengehäuses einsehbaren Anzeigeelement zusammen, beispielsweise mit einem an ihrer Oberfläche angebrachten Anzeigestift, der durch ein zugeordnetes Loch im Außengehäuse geführt ist und auf einfache Weise die Feststellung erlaubt, ob die Membran aktuell bis nahe zum Außengehäuse hin verformt ist. Alternativ könnte eine derartige Anzeigefunktion aber auch über eine elektronische Anzeige des Füllstandes, beispielsweise mittels LED, vorgesehen sein.

Die Kartusche ist in ihrer vorteilhaften Ausgestaltung ein vergleichsweise komplexes Bauteil mit einer Mehrzahl integrierter Funktionen, um die Handhabbarkeit besonders zu erleichtern. Um dies auf besonders effektive Weise zu nutzen, ist die Kartusche in vorteilhafter Ausgestaltung als Mehrwegprodukt ausgeführt, das nach dem Verbrauch der im Reservoir vorgehaltenen Flüssigkeit für einen erneuten Nutzungszyklus wieder befüllt werden kann. Vorteilhafterweise ist das Flüssigkeitsreservoir des Behälters dazu wiederbefüllbar.

Eine besonders hohe Funktionalität und gute Nutzbarkeit ist erreichbar, indem ein entsprechend ausgestalteter Verschluss- und Riegelmechanismus vorgesehen ist. Hierzu ist besonders vorteilhaft zur Öffnung bzw. zum Verschließen des Deckels des Außengehäuses ein Rotationsverschluss vorgesehen, über den auch die Freigabeventile des Kanalsystems und somit die medienseitige Verschaltung innerhalb des Kanalsystems steuerbar sind. Beispielhaft kann ein solchermaßen ausgestalteter Verschluss folgende Einstellmöglichkeiten bieten:
Wird der Verschluss leicht herausgezogen (dieser rastet dann vorzugsweise ein), so wird die Flüssigkeit aus den Linsenkammern nach außen gegeben, z. B. zum Entsorgen oder zum Spülen. Wird der Verschluss hingegen in eine Richtung, beispielsweise nach rechts, gedreht, so öffnet sich der Deckel; die Kartusche ist jedoch in dem Gehäuse blockiert und lässt sich nicht herauslösen. Wird der Rotationsverschluss hingegen um 180° in die Gegenrichtung, beispielsweise nach links, gedreht (dies sollte vorzugsweise nur bei offenem Deckel möglich sein), so kann die Kartusche leicht herausgelöst werden, um mit einer neuen, vollen Kartusche ersetzt zu werden. Bei vollständig offenem (beispielsweise herausgezogenem) Verschluss und Betätigung des Pumpknopfes kommt es zur Spülung der Linsen in den Kammern und gleichzeitigem Herauslassen und der Entsorgung der Flüssigkeit. In diesem Modus kann auch gezielt Flüssigkeit in die Hand des Nutzers gegeben werden, um z.B. seine Linsen manuell zu reiben.

In ganz besonders bevorzugter Ausgestaltung ist der Behälter, besonders bevorzugt dessen Kartusche, zudem für die Bereitstellung einer Vielzahl von Zusatzfunktionen ertüchtigt, die dem Nutzer eine verbesserte Nutzbarkeit und/oder einen noch weiter erhöhten Bedienkomfort zur Verfügung stellen sollen. Die Bereitstellung solcher Zusatzfunktionen erfolgt bevorzugt auf elektronischem Wege, und hierfür umfasst der Behälter, besonders bevorzugt dessen Kartusche, einen Energiespeicher für elektrische Energie. Als Energiespeicher kann dabei insbesondere eine Batterie oder ein Akku vorgesehen sein.

Als besonders bevorzugte Zusatzfunktion kann hierbei eine mechanische Reinigungsfunktion für die Kontaktlinsen zusätzlich zur Behandlung in der Pflegeflüssigkeit vorgesehen sein, wenn die Linsen sich in den Linsenkammern befinden. Durch diese Ausgestaltung können die Linsen einer kombinierten mechanischen und chemischen Behandlung unterworfen werden, so dass sich die Wirksamkeiten dieser Behandlungen synergistisch ergänzen. Hierzu ist der Energiespeicher vorteilhafterweise an eine Anzahl von dem oder den Kammerdeckeln zugeordneten Vibrationselementen angeschlossen. Gerade in Kombination mit den vorzugsweise vorgesehenen Membrandeckeln für die Linsenkammern können die Deckel somit als "Vibrationsmembranen" ausgeführt sein. Wenn diese bei eingelegter Linse vibrieren (ähnlich wie bei Vibrationszahnbürsten), so kann bereits bei Anwesenheit einer geringen Menge an Linsenflüssigkeit eine zuverlässige Säuberung der Linsen stattfinden. Die nach der Behandlung kontaminierte Flüssigkeit aus den Linsenkammern kann durch Pumpen und anschließendem Herausziehen des Rotationsverschlusses entsorgt werden. Dies kann auch bei einer elektrischen Ausführung so vorgesehen sein, dann bevorzugt im vollautomatischen Modus, bei dem der Nutzer dazu nichts tun muss. Die verunreinigte Flüssigkeit wird dann automatisch in das Zweitreservoir gepumpt; hiernach werden die Linsen zwecks Lagerung in ein frisches Flüssigkeitsbad getaucht.

Als weitere besonders bevorzugte Zusatzfunktionen, die durch Nutzung des Energiespeichers bereitgestellt werden können, sind - bevorzugt jeweils einzen oder in beliebiger Kombination miteinander - elektrisch angetriebene Pumpen zur Beförderung der Pflegeflüssigkeit im Kanalsystem, eine elektrische Anzeigeeinheit beispielsweise für Füllstand der Pflegeflüssigkeit im Reservoir und/oder in den Linsenkammern, Restzeit bis zur nächsten Pflegebehandlung oder dergleichen, oder die Anregung von elektroluminiszierenden Eigenschaften der Kontaktlinsen möglich. In besonders vorteilhafter Weiterbildung können auch UV-C-LEDs eingebaut sein, die eine Desinfektionswirkung auf die Linsen haben. Es könnte somit Licht der Art UV-A eingesetzt werden, welches für die Anregung von Lumineszenz günstig ist, und/oder besonders bevorzugt auch UV-Licht mit einer kürzeren Wellenlänge (UV-C) von beispielsweise etwa 200 nm. In besonders vorteilhafter, als eigenständig erfinderisch angesehener Weiterbildung sind zudem in den Linsenkammern, vorzugsweise an den Deckelmembranen, reflektierende Strukturen oder Elemente vorgesehen, die eine Homogenisierung des Lichts innerhalb der jeweiligen Linsenkammer ermöglichen oder begünstigen.

Die mit der Erfindung erzielten Vorteile bestehen insbesondere darin, dass durch die ermöglichte medienseitige kaskadenartige Hintereinanderschaltung von Flüssigkeitsreservoir, Linsenkammern und Auslass- oder Entsorgungsöffnung der Medientransport der Pflegeflüssigkeit gerade beim Spülvorgang konsequent von innen nach außen, also vom Flüssigkeitsreservoir weg hin zur Auslassöffnung, stattfindet und eine Rückwärtsströmung in das Reservoir hinein minimiert oder sogar ganz ausgeschlossen ist. Damit kann der Eintrag von Keimen von außen in das Reservoir hinein vermieden werden, so dass eine deutlich erhöhte Haltbarkeit und damit Lagerdauer der Flüssigkeit im Reservoir erreicht werden kann.

Ein Ausführungsbeispiel der Erfindung wird anhand einer Zeichnung näher erläutert. Darin zeigen:
- FIG. 1: einen Kontaktlinsen-Aufbewahrungs- und Reinigungsbehälter in perspektivischer Ansicht,
- FIG. 2: den Kontaktlinsen-Aufbewahrungs- und Reinigungsbehälter gemäß Figur 1 perspektivisch im Schnitt,
- FIG. 3: einen Ausschnitt aus Figur 2,
- FIG. 4: eine Kartusche des Kontaktlinsen-Aufbewahrungs- und Reinigungsbehälters in perspektivischer Ansicht,
- FIG. 5: die Kartusche gemäß Figur 4 perspektivisch im Schnitt,
- FIG. 6: die obere Kartuschenschale der Kartusche gemäß Figur 4 in einer Explosionsdarstellung von unten,
- FIG. 7: die obere Kartuschenschale der Kartusche gemäß Figur 4 in montiertem Zustand von unten,
- FIG. 8: eine alternative Ansicht der Kartusche gemäß Figur 4 im Schnitt,
- FIG. 9: eine alternative Ausführungsform einer Kartusche im Schnitt,
- FIG. 10, 11: jeweils einen Deckelverschluss des Behälters gem. FIG. 1 in Explosionsdarstellung,
- FIG. 12: eine alternative Ausführungsform eines Kontaktlinsen-Aufbewahrungs- und Reinigungsbehälters in perspektivischer Ansicht,
- FIG. 13: den Kontaktlinsen-Aufbewahrungs- und Reinigungsbehälter gemäß Figur 12 perspektivisch in Explosionsdarstellung,
- FIG. 14: ein Reservoirmodul des Kontaktlinsen - Aufbewahrungs- und Reinigungsbehälters gemäß Figur 12 perspektivisch in Explosionsdarstellung,
- FIG. 15-17: jeweils perspektivisch ein Basismodul des Kontaktlinsen - Aufbewahrungs- und Reinigungsbehälters gemäß Figur 12,
- FIG. 18: das Basismodul gem. FIG. 15 mit herausgenommenen Linsenkammern, und
- FIG. 19: die Linsenkammern des Basismoduls nach FIG. 15,
- FIG. 20: eine weitere alternative Ausführungsform eines Kontaktlinsen- Aufbewahrungs- und Reinigungsbehälters im Längsschnitt,
- FIG. 21: den Kontaktlinsen-Aufbewahrungs- und Reinigungsbehälter gemäß Figur 20 in geöffneter Position in Draufsicht,
- FIG. 22: den Kontaktlinsen-Aufbewahrungs- und Reinigungsbehälter gemäß Figur 20 in geöffneter Position in perspektivischer Ansicht,
- FIG. 23: den Kontaktlinsen-Aufbewahrungs- und Reinigungsbehälter gemäß Figur 20 mit abgeschrägten Stirnflächen in geöffneter Position in seitlicher Ansicht,
- FIG. 24: den Kontaktlinsen-Aufbewahrungs- und Reinigungsbehälter gemäß Figur 20 mit abgeschrägten Stirnflächen in geöffneter Position in Ansicht von unten,
- FIG. 25: einen alternativen Kontaktlinsen-Aufbewahrungs- und Reinigungsbehälter mit abgeschrägten Stirnflächen in geöffneter Position in seitlicher Ansicht,
- FIG. 26: eine weitere alternative Ausführungsform eines Kontaktlinsen- Aufbewahrungs- und Reinigungsbehälters in Ansicht von unten,
- FIG. 27: eine weitere alternative Ausführungsform eines Kontaktlinsen- Aufbewahrungs- und Reinigungsbehälters mit integrierter Pumpe im Längsschnitt,
- FIG. 28: den Behälter gem. Fig. 27 im Längsschnitt bei betätigter Pumpe,
- FIG. 29: eine alternative Ausführungsform eines Reservoirbeutels im Schnitt,
- FIG. 30: den Reservoirbeutel gem. Fig. 29 in Draufsicht,
- FIG. 31: den Reservoirbeutel gem. Fig. 29 in perspektivischer Ansicht,
- FIG. 32: den Reservoirbeutel gem. Fig. 29 in Explosionsdarstellung in seitlicher Ansicht, und
- FIG. 33: den Reservoirbeutel gem. Fig. 29 in Explosionsdarstellung in perspektivischer Ansicht.

Gleiche Teile sind in allen Figuren mit denselben Bezugszeichen versehen.

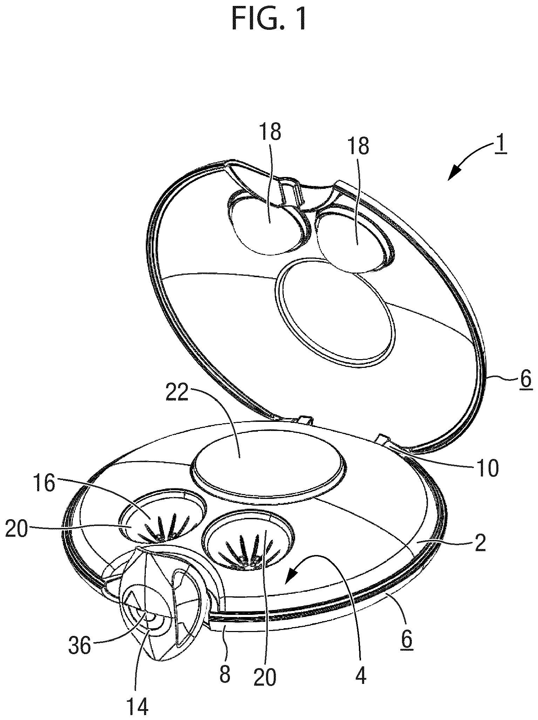

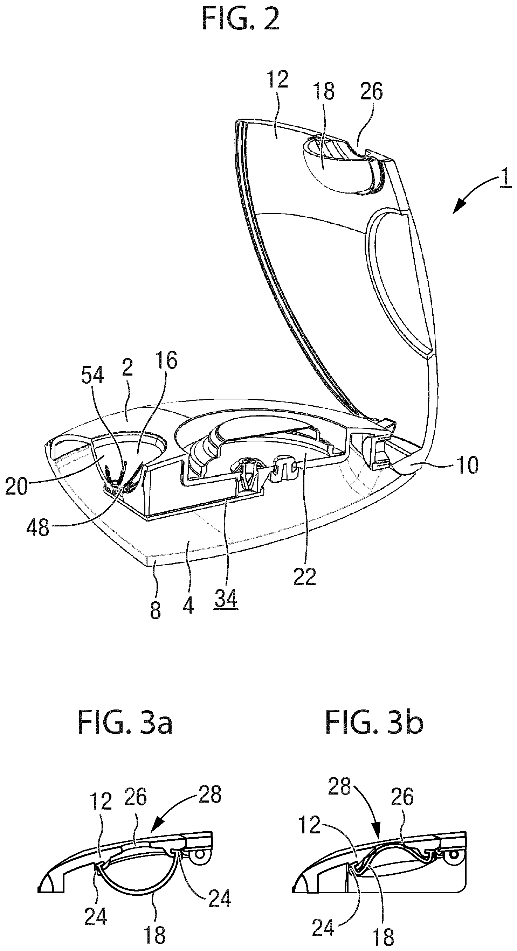

Der Kontaktlinsen-Aufbewahrungs- und Reinigungsbehälter 1, im Folgenden vereinfachend als "Behälter 1" bezeichnet, gemäß den Figuren 1, 2 dient zum vorübergehenden Verwahren von Kontaktlinsen und von einem Vorrat von zur Reinigung und zur Pflege der Kontaktlinsen vorgesehener Pflegeflüssigkeit. Dazu umfasst der Behälter 1 ein in eine austauschbare Kartusche 2 integriertes Reservoir 4 oder einen Tank mit einem Fassungsvermögen von bevorzugt und im Ausführungsbeispiel vorgesehen etwa 80 bis 100 ml, in dem die Reinigungs- oder Pflegeflüssigkeit vorgehalten werden kann. Die Kartusche 2 ist lösbar in einem Außengehäuse 6 angeordnet. Das Außengehäuse 6 ist im Ausführungsbeispiel durch einen unteren Boden 8 und durch einen mit diesem über ein Scharnier 10 klappbar verbundenen Gehäusedeckel 12 gebildet. In geschlossener Stellung ist der Gehäusedeckel 12 mittels eines drehbar ausgestalteten Deckelverschlusses 14 mit dem Boden 8 verriegelbar.

Zum Verwahren der Kontaktlinsen weist der Behälter 1 eine Anzahl von, im Ausführungsbeispiel zwei, Kontaktlinsenkammern 16 auf. Die Kontaktlinsenkammern 16, die im bevorzugten Ausführungsbeispiel jeweils ein Innenvolumen und somit Fassungsvermögen von etwa 1,5 ml aufweisen, werden jeweils durch einen Kammerdeckel 18 und durch einen zugeordneten Kammerboden 20 gebildet, wobei der Kammerboden 20 in geschlossenem Zustand durch den ihm zugeordneten Kammerdeckel 18 verschließbar ist. Der Kammerboden 20 ist dabei jeweils durch eine Vertiefung in der Oberfläche der Kartusche 2 gebildet, wobei der jeweils zugeordnete Kammerdeckel 18 am Gehäusedeckel 12 des Außengehäuses 6 angeordnet ist. Durch Öffnen oder Schließen des Außengehäuses 6 werden somit die Kontaktlinsenkammern 16 gleichermaßen und gleichzeitig geöffnet bzw. geschlossen.

Bei Verwahren der Kontaktlinsen in den Kontaktlinsenkammern 16 ist vorgesehen, dass diese mit Pflegeflüssigkeit aus dem Reservoir 4 befüllt werden, so dass die Kontaktlinsen feucht gehalten und ggf. gereinigt und/oder desinfiziert werden. Dazu ist in die Kartusche 2 eine manuell in der Art einer Druckpumpe bedienbare, im Ausführungsbeispiel als Faltenbalg ausgeführte Pumpvorrichtung 22 integriert, mittels derer Flüssigkeit aus dem Reservoir 4 in die Kontaktlinsenkammern 16 gefördert werden kann.

Wie insbesondere den ausschnittsweise vergrößerten Darstellungen in Figur 3 a,b entnehmbar ist, ist jeder Kammerdeckel 18 als verformbare Membran ausgeführt. Die Membran bildet dabei an ihrem umlaufenden, den Kontakt mit der Kartuschenoberfläche herstellenden Rand eine Dichtlippe 24 aus. In Figur 3a ist die Membran im entspannten Ruhezustand gezeigt. Beim Befüllen der jeweiligen Kontaktlinsenkammer 16 mit Flüssigkeit baut sich entsprechend Druck in der Linsenkammer auf, so dass die Membran nach oben zum Außengehäuse 6 hin nachgibt und Platz für die einströmende Flüssigkeit freigibt. Dieser Zustand ist in Figur 3b gezeigt. Gleichzeitig wird dabei Druck auf die seitliche Dichtlippe 24 ausgeübt, so dass sich der Kontakt mit der Oberfläche der Kartusche 2 und damit die Dichtungswirkung verstärkt. Am Außengehäuse 6 sind die Kammerdeckel 18 über jeweils einen am Außengehäuse 6 angeordneten Trägerring mit Hinterschnitt befestigt, auf den die jeweilige Membran einfach aufgesteckt werden kann. Die beiden die Kammerdeckel 18 bildenden Membranen sind dabei gleichartig und rotationssymmetrisch ausgeführt, so dass der Benutzer beim Auswechseln keine nennenswerte Rücksicht auf Ausrichtung oder Kammerseite nehmen muss.

Im Ausführungsbeispiel wird die Verformbarkeit der Membran auch zur Bereitstellung einer Indikatorfunktion genutzt, die dem Benutzer ermöglichen soll, festzustellen, ob die jeweilige Kontaktlinsenkammer gerade mit Pflegeflüssigkeit befüllt ist. Dazu ist im Gehäusedeckel 12 oberhalb der jeweiligen Membran ein Sichtfenster 26 oder eine Durchbrechung angeordnet. Über das Sichtfenster 26 ist für den Benutzer von außen erkennbar, ob die darunterliegende Membran nach oben hin ausgelenkt und die Kontaktlinsenkammer 16 dementsprechend befüllt ist. Die Erkennbarkeit kann dabei beispielsweise durch eine geeignet auf der Oberseite der Membran angebrachte Markierung und/oder durch einen an ihrer Oberfläche angebrachten Anzeigestift verstärkt sein. Diese Elemente einschließlich des Sichtfensters 26 bilden somit ein von außerhalb des Außengehäuses 6 einsehbares Anzeigeelement 28 für den Befüllungszustand der jeweiligen Kontaktlinsenkammer 16.

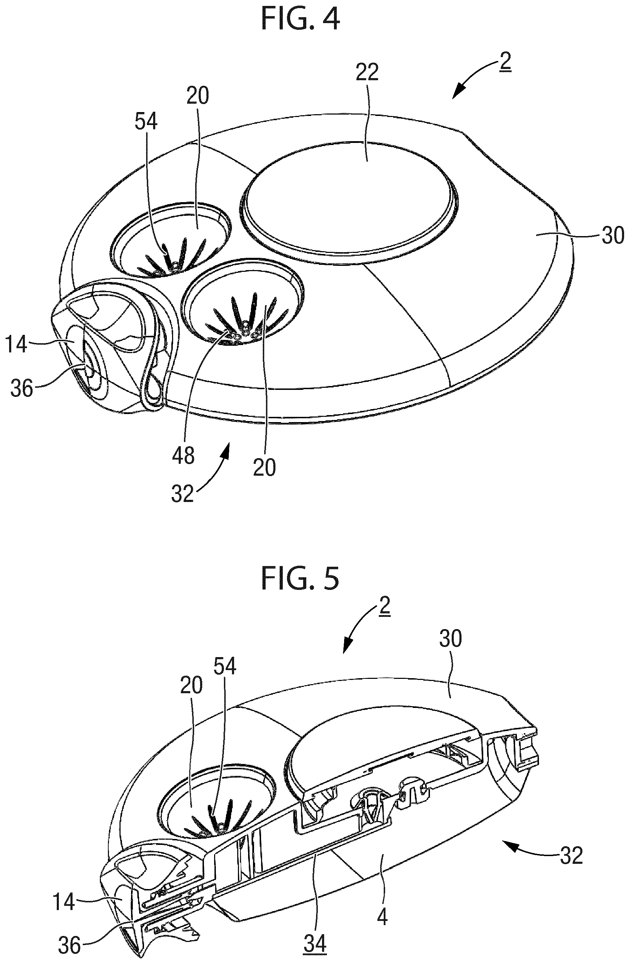

Die im Außengehäuse 6 angeordnete Kartusche 2, die in Figur 4 perspektivisch in seitlicher Ansicht und in Figur 5 perspektivisch im Schnitt gezeigt ist und deren Ausgestaltung als eigenständig erfinderisch angesehen wird, umfasst eine obere Kartuschenschale 30 und eine untere Kartuschenschale 32 auf, die an ihrem umlaufenden Rand zur Bildung des Kartuschengehäuses miteinander verschweißt sind. Die Kartuschenschale 30 ist bevorzugt als Spritzgussteil aus Polypropylen (PP) oder Polyethylen (PE) hergestellt. Demgegenüber ist die Kartuschenschale 32 als vergleichsweise elastisches Bauteil ausgeführt, so dass sie sich zusammenziehen kann. Als Material dafür ist bevorzugt ein TPE vorgesehen, aber auch Silikon oder ein dehnbares LDPE ist denkbar. Diese Teile werden allerdings nicht im Spritzguss hergestellt. Alternativ könnte auch ein rigider Ring vorgesehen sein, der spritzgegossen werden sollte und in der Mitte aus einem elastischen Material bestehen könnte. Dies könnte den Schweißprozess erleichtern. Als integrierte Elemente weist die Kartusche 2 das Flüssigkeits-Reservoir 4 und die Pumpvorrichtung 22 auf, und der Deckelverschluss 14 ist ebenfalls an der Kartusche 2 angebracht. Des Weiteren ist in die Kartusche 2 ein Kanalsystem 34 integriert, über das die Flüssigkeit mittels der Pumpvorrichtung 22 vom Reservoir 4 in die Kontaktlinsenkammern 16 eingebracht werden kann.

Die Kartusche 2 und mit dieser der Behälter 1 sind gezielt für eine besonders lange Haltbarkeit der im Reservoir 4 vorgehaltenen Reinigungs- oder Pflegeflüssigkeit ausgelegt. Dabei ist insbesondere berücksichtigt, dass die Haltbarkeit der Flüssigkeit durch eindringende Keimlasten begrenzt werden könnte. Um dem entgegenzuwirken, ist das Kanalsystem 34 derart ausgelegt, dass die Flüssigkeit mittels der Pumpvorrichtung 22 über das Kanalsystem 34 von dem Flüssigkeitsreservoir 4 aus über die Kontaktlinsenkammern 16 einer im Deckelverschluss 14 angebrachten Auslassöffnung 36 zugeführt werden kann. Somit kann flüssigkeitsseitig eine kaskadenartige Hintereinanderschaltung der Elemente Reservoir 4, Kontaktlinsenkammer 16 und Auslassöffnung 36 hergestellt werden, so dass die Flüssigkeit lediglich in der Richtung auswärts zur Auslassöffnung 36 strömen kann und eine Rückwärtsströmung in das Reservoir 4 hinein minimiert oder nahezu ausgeschlossen werden kann. Das Spülen der Linsen in den Kammern bzw. das Herauslassen der verunreinigten Flüssigkeit aus den Kammern beim Herausziehen des Deckelverschlusses wird durch die als Deckelmembranen ausgestalteten Kammerdeckel ebenfalls begünstigt. Diese gehen nämlich in die deflektierte Ausgangsposition zurück, sobald es eine Druckentlastung gibt, beispielsweise durch Öffnung des äußeren Kanals durch das Herausziehen des Verschlusses.

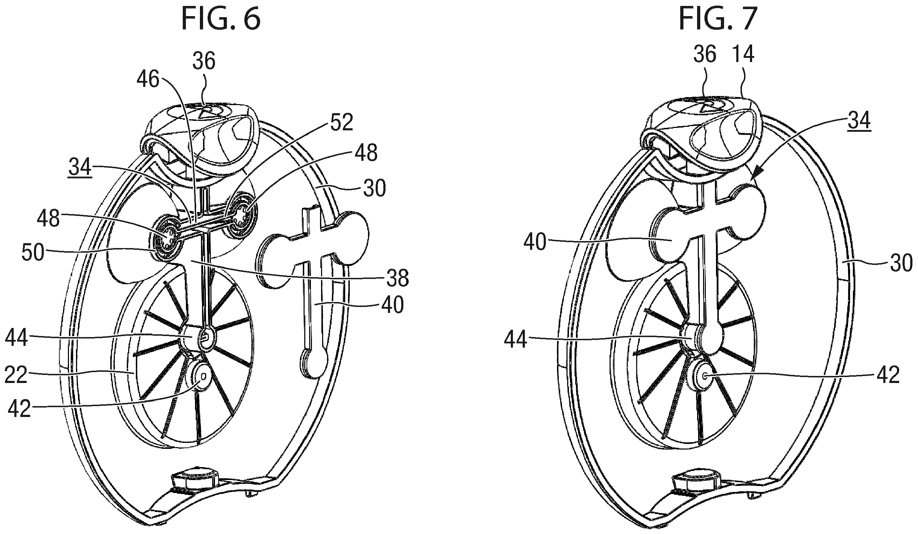

Zur Verdeutlichung dieser Auslegung ist die obere Kartuschenschale 30 in FIG. 6 (als Explosionsdarstellung) und in FIG. 7 (montiert) in einer Ansicht von unten dargestellt. Wie den Figuren 6, 7 entnehmbar ist, weist die als Spritzgussteil ausgeführte obere Kartuschenschale 30 an ihrer unteren, dem Innenraum und damit dem Reservoir 4 zugewandten Seite ein angeformtes Kanalstück 38 auf, das zur Bildung des Kanalsystems 34 mit einer aufgeschweißten Kanalplatte 40 verbunden ist. Wie in diesen Darstellungen erkennbar ist, ist die Pumpvorrichtung 22 flüssigkeitsseitig über ein Schirmventil 42 mit dem Reservoir 4 verbunden. Dieses Schirmventil 42 soll einen Überdruck im Pumpenraum verhindern. Es öffnet im Überdruckfall in das Reservoir: sobald der Druck in der Pumpe zu hoch wird entsteht hier ein "Kurzschluss". Da die Flüssigkeit aus dem Kanalsystem jedoch niemals wieder in den Pumpenraum gelangt, so führt dieser Kurzschluss zu keiner Verunreinigung der Reservoirflüssigkeit. Des Weiteren ist die Pumpvorrichtung 22 über ein Rückschlagventil 44 mit dem Kanalsystem 34 verbunden, wobei das Rückschlagventil 44 ebenfalls in der Art einer unidirektionalen Ausführung lediglich ein Ausströmen von Flüssigkeit aus dem Innenraum der Pumpvorrichtung 22 in das Kanalsystem 34, nicht aber ein Rückströmen von Flüssigkeit aus dem Kanalsystem 34 in die Pumpvorrichtung 22 hinein erlaubt.

Um die Erzeugung zu hoher Drücke in den Kontaktlinsenkammern 16 bei Betätigung der Pumpvorrichtung 22 auszuschließen, ist diese zudem mit einem Überdruckventil abgesichert. Dieses kann durch das Rückschlagventil 44 gebildet sein.

Bei der Nutzung wird zunächst Reinigungsflüssigkeit aus der Kartusche 4 in das Reservoir der Pumpvorrichtung 22 eingeleitet. Von dort gelangt die Reinigungsflüssigkeit über das Kanalsystem 34 in die Kontaktlinsenkammern 16 und umspült und reinigt die dort befindlichen Linsen. Nach Beendigung des Reinigungsvorgangs und bei oder vor der Entnahme der Linsen aus den Kontaktlinsenkammern 16 entweicht dann die verbrauchte Reinigungsflüssigkeit durch die Auslassöffnung 36.

Das Kanalsystem 34 umfasst dabei einen mittig oder zentral angeordneten, in die Kontaktlinsenkammern 16 führenden Zuströmkanal 46. Dieser mündet über eine Anzahl - im Ausführungsbeispiel sechs - von im jeweiligen Kammerboden 20 angeordneten Einströmlöchern 48 in das Innere der jeweiligen Kontaktlinsenkammer 16. In Auslass- oder Abströmrichtung ist die jeweilige Kontaktlinsenkammer 16 über ebenfalls im Kammerboden 20 angeordnete Ausströmlöcher 50 und einen neben dem Zuströmkanal 46 platzierten Abströmkanal 52 mit dem Deckelverschluss 14 verbunden. Um dabei auch bei in die Kontaktlinsenkammern 16 eingelegten Kontaktlinsen einen zuverlässigen Medienaustausch zu gewährleisten und ein unbeabsichtigtes Verschließen der Ein- und/oder Ausströmlöcher 48, 50 durch die Kontaktlinsen zu vermeiden, ist der Kammerboden 20 mit einer Anzahl von Trägerstegen 54 versehen, auf denen die Kontaktlinse beabstandet von den Ein- bzw. Ausströmlöchern 48, 50 aufliegt.

Die Führung der Flüssigkeit erfolgt dabei - neben anderen Funktionen - mittels des Deckelverschlusses 14. Dieser ist einerseits als Rotationsverschluss zur Öffnung bzw. zum Verschließen des Gehäusedeckels 12 des Außengehäuses 6 ausgeführt. Zusätzlich werden über den Deckelverschluss 14 aber auch die - nicht näher dargestellten - integrierten Freigabeventile des Kanalsystems 34 und somit die medienseitige Verschaltung innerhalb des Kanalsystems 34 gesteuert. Im Ausführungsbeispiel sind dabei folgende Einstellmöglichkeiten vorgesehen, wobei selbstverständlich auch andere konkrete Ausgestaltungen möglich sind:
Wenn der Deckelverschluss 14 leicht herausgezogen wird (und dabei vorzugsweise einrastet), wird die Auslassöffnung 36 freigegeben und damit die Flüssigkeit aus den Kontaktlinsenkammern 16 nach außen gegeben, z. B. zum Entsorgen oder zum Spülen. Wird der Deckelverschluss 14 hingegen in eine Richtung, beispielsweise nach rechts, gedreht, so öffnet sich der Gehäusedeckel 12; die Kartusche 2 ist jedoch im Außengehäuse 6 blockiert und lässt sich nicht herauslösen.

Die Kartusche ist im Gehäuse durch den Verschluss blockiert, solange dieser nicht um 180° nach links gedreht wird (bei offenem Deckel). Die Drehung des Verschlusses ist für die Flüssigkeitsausgabe jedoch irrelevant. Der Nutzer kann zu Beginn den Verschluss jedoch lediglich herausziehen; ein Drehen ist nur möglich, wenn der Verschluss herausgezogen ist.

Wird der Deckelverschluss 14 hingegen um 180° in die Gegenrichtung, beispielsweise nach links, gedreht (dies sollte vorzugsweise nur bei offenem Deckel möglich sein), so kann die Kartusche 2 leicht herausgelöst werden, um durch eine neue, volle Kartusche 2 ersetzt zu werden. Bei vollständig offenem (beispielsweise herausgezogenem) Deckelverschluss 14 und Betätigung des Knopfes der Pumpvorrichtung 22 kommt es zur Spülung der Linsen in den Kammern und gleichzeitigem Herauslassen und der Entsorgung der Flüssigkeit. In diesem Modus kann auch gezielt Flüssigkeit in die Hand des Nutzers gegeben werden, um z.B. seine Linsen manuell zu reiben.

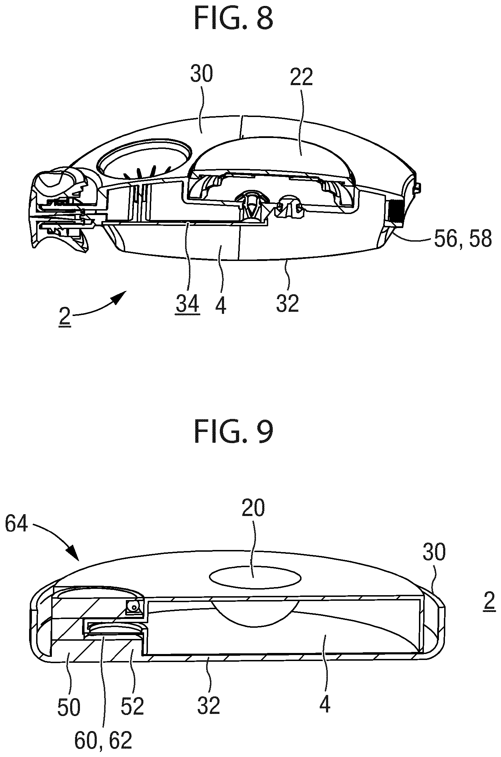

Wie der alternativen Ansicht der Kartusche 2 in Figur 8 entnehmbar ist, ist das Reservoir 4 wiederbefüllbar ausgeführt. Dazu sind endseitig am der Auslassöffnung 36 und dem Drehverschluss 14 gegenüberliegenden Seite des Kartuschenkörpers eine Anzahl von Befüllöffnungen 56 vorgesehen, über die in der Art einer Betankung mittels einer geeignet ausgeführten Fülllanze die Einbringung der Flüssigkeit in das Reservoir ermöglicht ist. Jeder Befüllöffnung 56 ist ein Verschlussstopfen 58 zugeordnet, mit dem die jeweilige Öffnung nach der Befüllung verschlossen werden kann. Grundsätzlich könnte dabei eine einzige Befüllöffnung 56 vorgesehen sein, über die die Kartusche 2 in der Art eines Mehrwegprodukts mehrfach und an sich unbegrenzt oft wiederbefüllt werden kann. Alternativ können die Systeme aus Befüllöffnung 56 und zugeordnetem Verschlusstopfen 58 aber auch für lediglich einmaligen Gebrauch ausgeführt sein, so dass die Anzahl der möglichen Wiederbefüll-Vorgänge durch die Anzahl der noch zur Verfügung stehenden "unverbrauchten" Befüllöffnungen 56 begrenzt ist.

In einer besonders bevorzugten und als eigenständig erfinderisch angesehenen Ausführungsform kann die Kartusche 2 und mit dieser der Behälter 1 auch mit elektrisch aktivierbaren oder angesteuerten Aggregaten zur Bereitstellung weiterer Zusatzfunktionen ausgerüstet sein. In einem hierfür geeigneten Ausführungsbeispiel, wie es für die Kartusche 2 in Figur 9 im Schnitt dargestellt ist, umfasst diese einen Energiespeicher 60 für elektrische Energie. Dieser kann als Batterie ausgeführt sein, im dargestellten bevorzugten Ausführungsbeispiel ist als Energiespeicher 60 aber ein Akkumulator 62 vorgesehen, der zum Zweck einer Wiederaufladung über eine in die Kartusche 2 integrierte Ladebuchse 64 an eine externe Stromversorgung anschließbar und über diese wiederaufladbar ist. Insbesondere kann eine Möglichkeit zur Wiederaufladung über eine Induktionsspule, intergiert oder auch extern, vorgesehen sein.

Die Nutzung des Energiespeichers 60 kann für eine Vielzahl möglicher Anwendungen vorgesehen sein. Als besonders bevorzugte Zusatzfunktion kann hierbei eine mechanische Reinigungsfunktion für die Kontaktlinsen zusätzlich zur Behandlung in der Pflegeflüssigkeit vorgesehen sein, wenn die Linsen sich in den Kontaktlinsenkammern 16 befinden. Durch diese Ausgestaltung können die Linsen einer kombinierten mechanischen und chemischen Behandlung unterworfen werden, so dass sich die Wirksamkeiten dieser Behandlungen synergistisch ergänzen. Hierzu kann der Energiespeicher 60 an eine Anzahl von den Kammerdeckeln 18 zugeordneten Vibrationselementen angeschlossen sein. Die als Membrandeckel ausgeführten Kammerdeckel 18 der Kontaktlinsenkammern 16 sind dementsprechend als "Vibrationsmembranen" ausgeführt. Wenn diese bei eingelegter Linse vibrieren, kann bereits bei Anwesenheit einer geringen Menge an Linsenflüssigkeit eine zuverlässige Säuberung der Linsen stattfinden.

Weitere Zusatzfunktionen, die durch Nutzung des Energiespeichers 60 bereitgestellt werden können, sind beispielsweise elektrisch angetriebene Pumpen zur Beförderung der Pflegeflüssigkeit im Kanalsystem 34, eine elektrische Anzeigeeinheit beispielsweise für Füllstand der Pflegeflüssigkeit im Reservoir 4 und/oder in den Linsenkammern, Restzeit bis zur nächsten Pflegebehandlung oder dergleichen, oder die Anregung von elektroluminiszierenden Eigenschaften der Kontaktlinsen, z.B. durch die Erzeugung von elektromagnetischen Wellen (in dem Fall über UV-Licht).

Ein Ausführungsbeispiel dafür ist eine über den Energiespeicher 60 antreibbare, in die Kartusche 2 integrierte Pumpe.

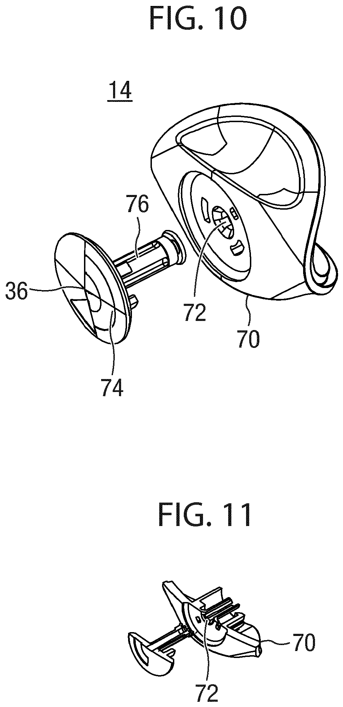

Der Deckelverschluss 14 ist im besonders bevorzugten Ausführungsbeispiel, wie dies den Explosionsdarstellungen in den Figs. 10 und 11 entnehmbar ist, seinerseits mehrteilig ausgeführt. Als Komponenten umfasst er das äußere eigentliche Bedienteil 70, das aus einem vergleichsweise harten Plastikmaterial gebildet ist und geeignete Ausformungen als Griffflächen zur Bedienung aufweist. In einen Aufnahmekanal 72 im Mittelbereich des Bedienteils 70 ist ein aus einem vergleichsweise weichen Plastikmaterial gebildetes Ventilteil 74 einsteckbar. Dieses weist ein Kanalstück 76 auf, das eingangsseitig mit dem Kanalsystem 34 in der Kartusche 2 verbindbar ist und ausgangsseitig die Auslassöffnung 36 bildet. Das in der Art einer Weichkomponente ausgeführte Kanalstück 76 ist dabei ähnlich einem Schnabelventil geformt, so dass nach der Beendigung des Ausspülvorgangs keine Flüssigkeit aus dem Inneren des Behälters mehr nachtropft.

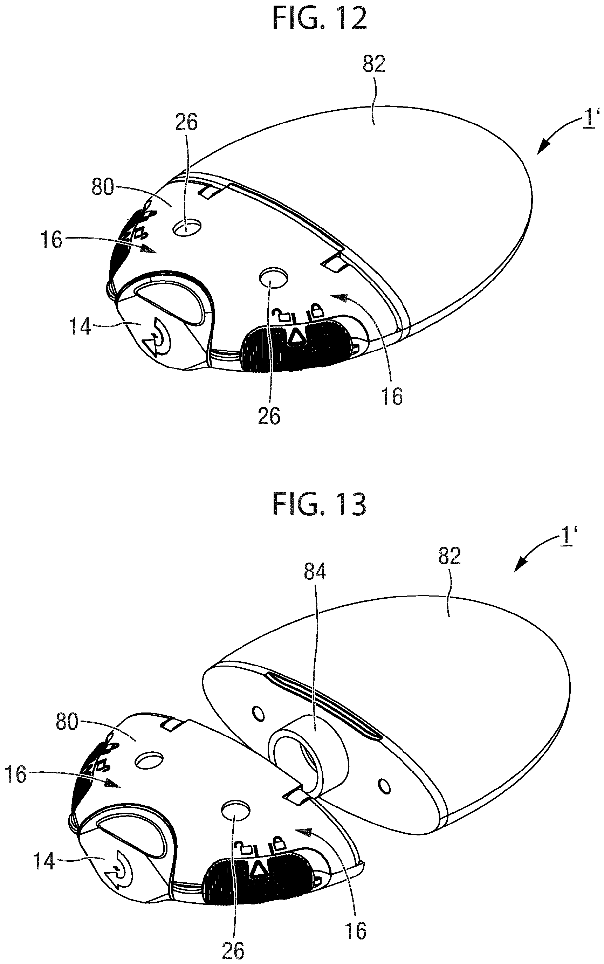

Eine alternative Ausführungsform eines erfindungsgemäßen Kontaktlinsen-Aufbewahrungs- und Reinigungsbehälters 1' ist in den Figs. 12 ff gezeigt. In dieser Variante umfasst der Kontaktlinsen-Aufbewahrungs- und Reinigungsbehälter 1' im Wesentlichen zwei Module, nämlich einerseits ein Basismodul 80 und andererseits ein Reservoirmodul 82. Wie der Gesamtansicht in FIG. 12 und der Explosionsdarstellung in FIG. 13 entnehmbar ist, sind die Module 80, 82 zusammensteckbar. Das in den Figs. 12, 13 mit geschlossenen Kontaktlinsenkammern 16 dargestellte Basismodul 80 umfasst dabei die wesentlichen, für die erfindungsgemäße Handhabung der Kontaktlinsen vorgesehenen Komponenten, nämlich beispielsweise die eigentlichen Kontaktlinsenkammern 16, den Deckelverschluss 14, das Kanalsystem 34, die Pumpvorrichtung 22 und dergleichen. Das Reservoirmodul 82 ist hingegen zur Aufnahme des Reservoirs 4 für die Reinigungsflüssigkeit vorgesehen. Die mechanische Verbindung zwischen den Modulen 80, 82 wird dabei durch eine Anzahl von Verbindungssteckern 84 hergestellt, die an jeweils einem der Module 80, 82 angeformt und in zugeordnete Steckmuffen im jeweils anderen Modul 80, 82 einsteckbar sind. Der oder die Verbindungsstecker 84 können dabei mit integrierten Verbindungskanälen zur Verbindung des Reservoirs 4 für die Reinigungsflüssigkeit mit dem Kanalsystem 34 im Basismodul 80 ausgestattet sein, so dass zusätzlich zur mechanischen Verbindung der Module 80, 82 miteinander auch die flüssigkeitsseitige Verbindung des Reservoirs 4 mit dem Kanalsystem 34 über die Verbindungsstecker 84 realisiert ist.

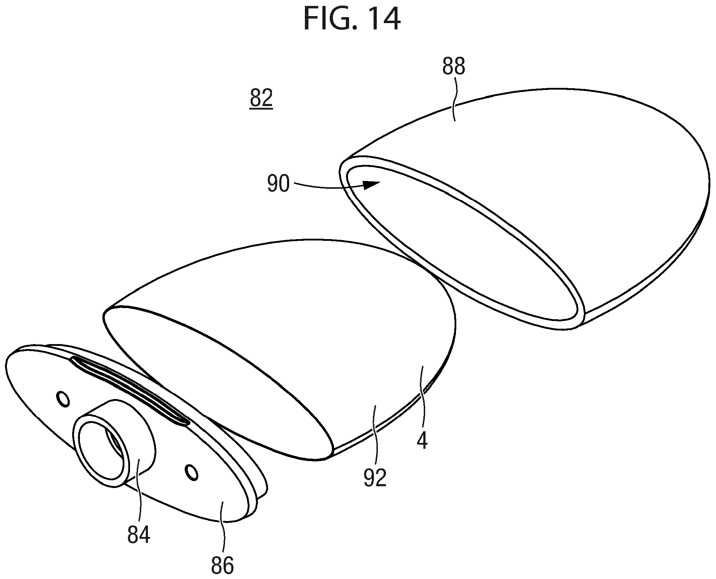

Das in FIG. 14 in Explosionsdarstellung gezeigte Reservoirmodul 82 ist dabei seinerseits mehrteilig aufgebaut und umfasst eine zur Herstellung der Verbindung mit dem Basismodul 80 vorgesehene und mit den Verbindungssteckern 84 versehene Frontplatte 86 sowie eine mit der Frontplatte zur Bildung eines Außengehäuses verbindbare Gehäuseschale 88. In das von Frontplatte 86 und Gehäuseschale 88 in montiertem Zustand gebildete Innenvolumen 90 ist das Reservoir 4 für die Reinigungsflüssigkeit einbringbar. Das Reservoir kann dabei durch die Kartusche 2 gemäß der vorgenannten Ausführungsform gebildet sein; alternativ und in als eigenständig erfinderisch angesehener Ausführungsform ist im gezeigten Beispiel zur Bildung des Reservoirs 4 aber ein Reservoirbeutel 92 mit weicher, verformbarer Außenhaut vorgesehen. Diese Bauweise erlaubt einen besonders leichten Austausch des Reservoirs 4, zum Ersatz der Reinigungsflüssigkeit, bei besonders hoher Flexibilität der Aufnahmemenge des Reservoirs 4. Die vergleichsweise stabil ausgeführte Gehäuseschale 88 schützt dabei den an sich mit weicher Außenhaut ausgeführten Reservoirbeutel 92 vor mechanischer Beschädigung.

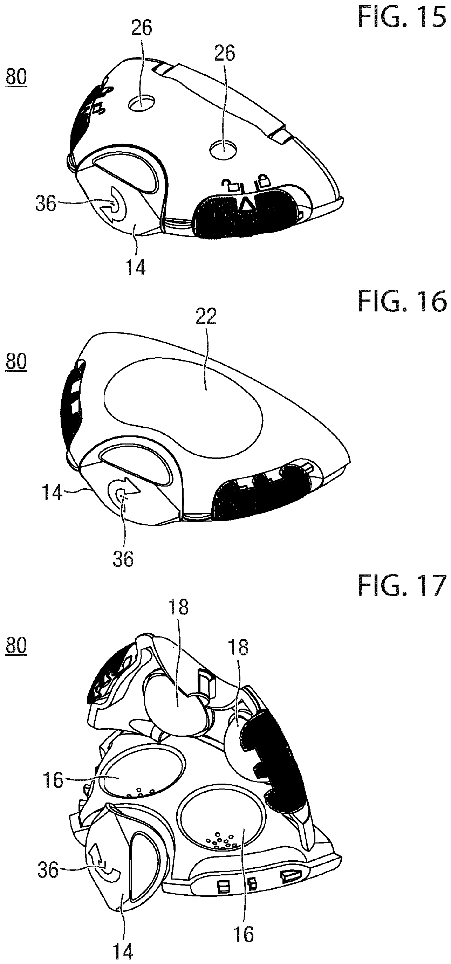

Das in FIG. 15 perspektivisch in oberer Ansicht, in FIG. 16 perspektivisch in unterer Ansicht und in FIG. 17 im Zustand mit geöffneten Kontaktlinsenkammern 16 gezeigte Basismodul 80 umfasst wie oben dargestellt die Komponenten Kontaktlinsenkammern 16, Pumpenvorrichtung 22, Deckelverschluss 14 sowie das Kanalsystem 34.

Der Behälter gemäß dieses Ausführungsbeispiels gliedert sich somit nun in zwei Teile, dem als Basismodul 80 vorgesehenen Vorderteil, der die Kontaktlinsenkammern 16, den Drehverschluss 14, die expandierenden Deckelmembranen mit dem zugehörigen Deckel, die Unterschale (zur Aufnahme der Linsenkammern 16), die Pumpe 22 und die Ventile, sowie evtl. noch zusätzliche Verriegelungsmechanismen enthält (in den Bildern als "Schieber" dargestellt).

Das als Reservoirmodul 82 ausgestaltete Hinterteil ist in diesem Ausführungsbeispiel nur für die Kartusche 4 vorgesehen, die auf diese Weise äußerst günstig produziert werden kann. Die Kartusche 4 besteht dabei im Wesentlichen aus einem weichen Beutel, der an einer harten Komponente angeschweißt wird; diese weist einen als "Konnektor" bezeichneten Anschluss auf, der mit dem Vorderteil eine dichte Verbindung eingehen kann (z.B. durch Einschrauben oder durch Zusammenstecken). Auf diese Weise wird die Verbindung zwischen Kartusche und Vorderteil hergestellt.

Ähnlich wie bei einem Infusionsbeutel zieht sich nun auch der hiesige Beutel zusammen, sobald Flüssigkeit aus diesem entnommen wird. Der Beutel ist von einer harten Schale bedeckt, die analog dem Vorderteil hochqualitativ hergestellt werden kann. Das Auswechseln der Kartusche geschieht in diesem Fall durch Herausnehmen des leeren Beutels aus der Kartuschenschale und durch das Ersetzen mit einem vollen Beutel. Dieser Beutel bildet mit der darüberliegenden harten Komponente eine Einheit.

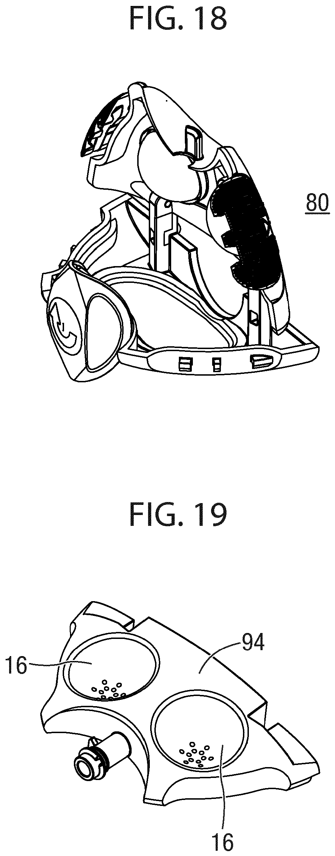

Die Kontaktlinsenkammern 16 bzw. deren untere Gehäusehälfte werden durch ein entsprechend ausgestaltetes Formteil 92 gebildet, das in das Basismodul 80 eingelegt und aus diesem herausnehmbar ist. In FIG. 18 ist der Vorderteil oder das Basismodul 80 des Behälters in einem Zustand gezeigt, bei dem das Reservoirmodul 82 abgekoppelt ist und das Formteil 94 herausgenommen wurden. Das Basismodul 80 bildet in diesem Fall eine leere Schale. Das herausgenommene Formteil 94 ist in FIG. 19 gezeigt.

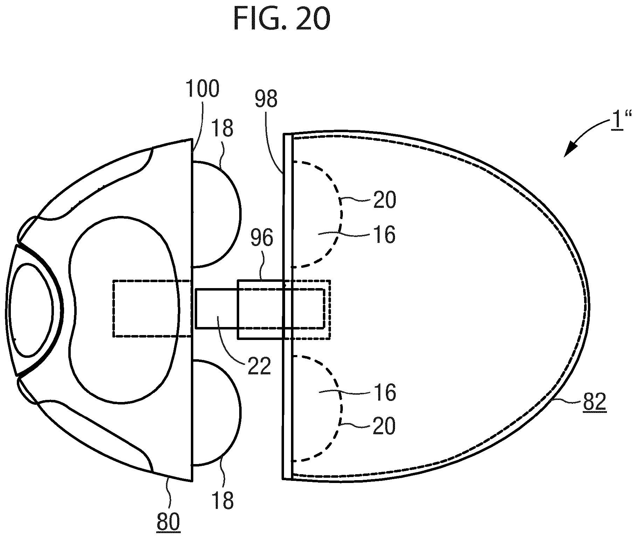

Eine weitere alternative Ausführungsform eines erfindungsgemäßen Kontaktlinsen-Aufbewahrungs- und Reinigungsbehälters 1" ist in den Figs. 20 ff gezeigt. In dieser Variante ist ebenfalls eine Aufteilung in im Wesentlichen zwei Module, nämlich einerseits das Basismodul 80 und andererseits das Reservoirmodul 82, vorgesehen, wobei in dieser Variante in als eigenständig erfinderisch angesehener Ausführung das Basisteil 80 in der Art eines rotierbaren Kopfteils ausgeführt und relativ zum Reservoirmodul 82 verdrehbar ausgeführt ist. Dazu ist, wie dies der Schnittzeichnung in Fig. 20 entnehmbar ist, das Basisteil 80 über einen Verbindungsflansch 96 mit dem Reservoirteil 82 verbunden. Der Verbindungsflansch 96 ist mit einem intergierten Kanal zum Austausch der Linsenflüssigkeit zwischen Reservoirmodul 82 und Basisteil 80 versehen. In diesen Kanal ist dabei auch die in diesem Ausführungsbeispiel als Kolbenpumpe ausgestaltete Pumpeneinheit 22 integriert. Das Basisteil 80 ist in Längsrichtung verschiebbar auf dem Verbindungsflansch 96 angeordnet und grundsätzlich ähnlich wie der Deckelverschluss 14 bedienbar: bei "herausgezogenem", d. h. vom Reservoirmodul 82 weg verschobenem Basisteil 80 kommt dieses in eine Position auf dem Verbindungsflansch 96, in der es auf diesem in der Art einer Wellenlagerung drehbar ist. Die Kontaktlinsenkammern 16 werden in dieser eigenständig erfinderischen Ausführung im Verbindungsbereich der beiden Module von deren Stirnflächen 98,100 gebildet, wobei der Kammerboden 20 jeder Linsenkammer 16 in der Art einer Ausformung in die Stirnfläche 98 des Reservoirmoduls 82 intergiert und der jeweils als Membran ausgeführt Kammerdeckel 18 an der Stirnfläche 100 des Basismoduls 80 angebracht ist. Die Linsenkammern 16 werden dabei somit dadurch verschlossen, dass das Basismodul 80 mit seiner Stirnfläche 100 auf dem Verbindungsflansch 96 bis vollständig hin zur Stirnfläche 98 des Reservoirmoduls 82 verschoben wird.

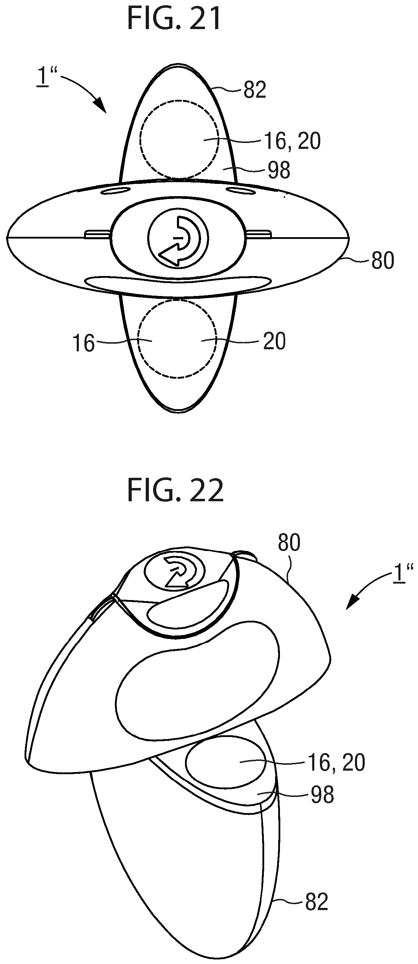

Um die Linsen aus den Linsenkammern 16 entnehmen zu können, muss in dieser Ausführungsform somit das Basismodul 80 auf dem Verbindungsflansch 96 vom Reservoirmodul 82 weg verschoben und anschließend auf dem Verbindungsflansch 96 um einen Öffnungswinkel, im Ausführungsbeispiel und besonders bevorzugt 90°, verdreht werden. Diese geöffnete Position zur Entnahme der Linsen ist in den Ansichten gemäß Fig. 21 (Draufsicht) und Fig. 22 (perspektivische Ansicht) gezeigt.

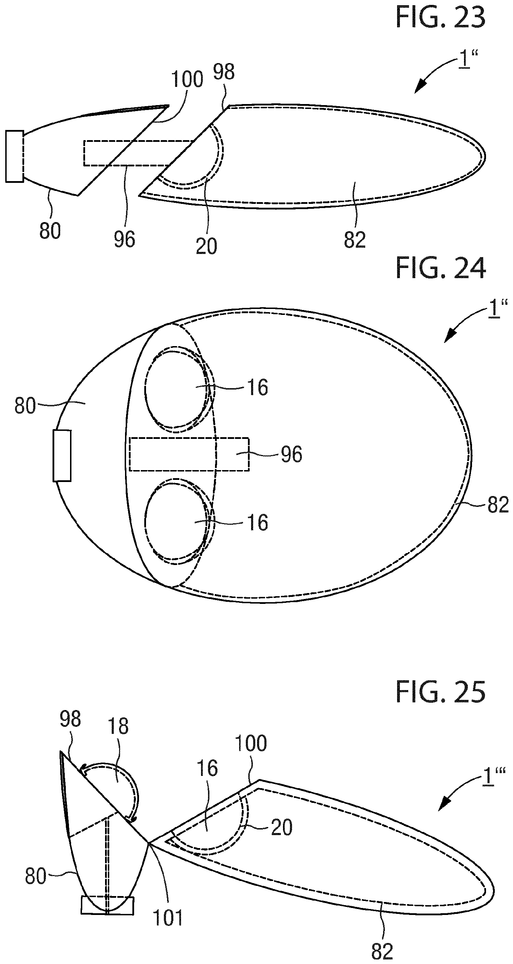

In besonders vorteilhafter Weiterbildung ist diese alternative Variante des Kontaktlinsen-Aufbewahrungs- und Reinigungsbehälters 1" mit abgeschrägten Stirnflächen 98, 100 von Basismodul 80 und Reservoirmodul 82 ausgeführt, wie dies mit geöffneten Linsenkammern 16 in seitlicher Ansicht in Fig. 23 und in Ansicht von unten in Fig. 24 gezeigt ist. Diese besonders bevorzugte Ausführung hat den wesentlichen Vorteil, dass dadurch die Zugriffsfläche der Linsenkammern 16 vergrößert und somit die Entnahme der Linsen erleichtert ist, wobei zudem das Volumen der Linsenkammern 16 vergleichsweise vergrößert ist, so dass diese auch bei vergleichsweise schmal oder schlank gehaltenen Stirnflächen 98, 100 in diese integriert sein können.

Alternativ zu der in den Figs. 23, 24 gezeigten Variante, bei der Basismodul 80 und Reservoirmodul 82 in Längsrichtung relativ zueinander verschiebbar sind, wobei bei auseinandergezogener Position die Linsenkammern 16 frei gegeben und damit zugänglich gemacht werden können, kann gemäß der in Fig. 25 gezeigten Variante des Basismodul 80 auch über ein Scharnier 101 schwenk- oder klappbar mit dem Reservoirmodul 82 verbunden sein. In Fig. 25 ist diese Variante im geöffneten Zustand gezeigt, in dem die Linsenkammern 16 ebenfalls geöffnet und die darin befindlichen Linsen somit zugänglich sind.

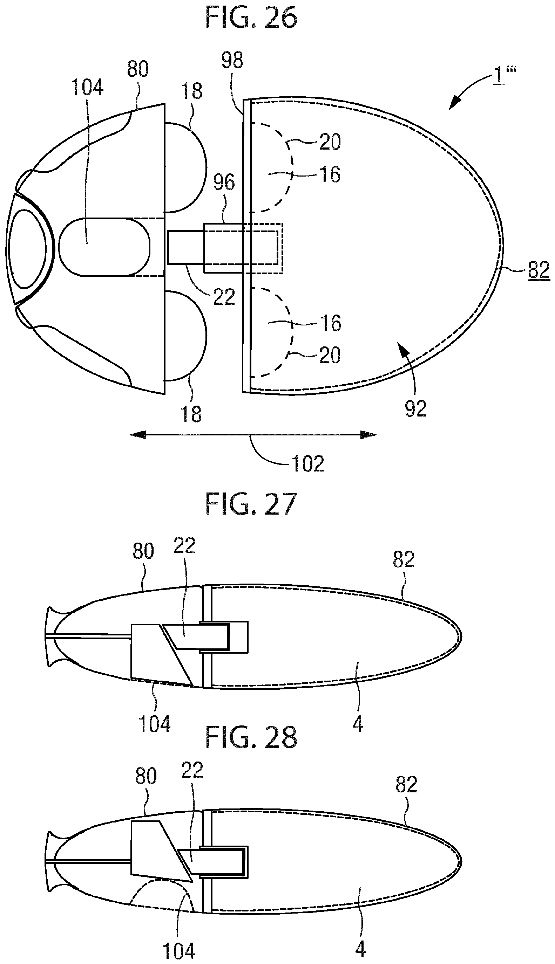

Eine noch weitere alternative Ausführungsform eines erfindungsgemäßen Kontaktlinsen-Aufbewahrungs- und Reinigungsbehälters 1‴ ist in den Figs. 26 ff gezeigt. Auch in dieser Variante ist eine Aufteilung in im Wesentlichen zwei Module, nämlich einerseits das Basismodul 80 und andererseits das Reservoirmodul 82, vorgesehen, wobei das Basismodul 80 in dieser Variante zur Verbindung mit dem Reservoirmodul 82 auf den Verbindungsflansch 96 aufgesteckt oder auf diesem verdrehbar gelagert sein kann. Als eigenständig erfinderisch wird in dieser Ausführung angesehen, dass eine als Pumpvorrichtung 22 vorgesehene Kolbenpumpe in den Verbindungsflansch 96 und somit in das Reservoirmodul 82 oder den Reservoirbeutel 92 bzw. die Kartusche 2 integriert ist. Damit ist sichergestellt, dass bei vom Nutzer nicht wiederbefüllbarer Kartusche 2 nach jedem Kartuschenwechsel automatisch eine frische, unverbrauchte Pumpe bereitgestellt wird.

Der Pumpvorgang kann dabei, wie in der Draufsicht gem. Fig. 26 durch den Doppelpfeil 102 symbolisiert, durch Betätigung der Pumpvorrichtung 22 durch Zusammendrücken und Auseinanderziehen von Basis- und Reservoirmodul 80, 82 oder alternativ durch Betätigung eines im Gehäuse angeordneten, auf die Kolbenpumpe wirkenden Pumpknopfs 104 durchgeführt werden. Eine Variante des Kontaktlinsen-Aufbewahrungs- und Reinigungsbehälters 1‴ mit einem solchen Pumpknopf 104 ist im Längsschnitt in den Figs. 27 (nicht komprimierte Pumpe) und 28 (komprimierte Pumpe) gezeigt. Die Betätigung der Pumpe 22 kann dabei wie dargestellt über den Pumpknopf 104 oder aber auch durch ein Langloch im Basismodul 80 oder durch seitlich an dieser angeordnete Pumpgriffe erfolgen. Diese bevorzugte Ausführung hat den Vorteil, dass der Nutzer auf diese Weise mit nur einer Hand Flüssigkeit in seine andere Hand geben kann, um die Linsen zu reiben. Beim Auseinanderziehen bzw. Zusammenschieben von Basis und Reservoir bräuchte man hingegen zwei Hände hierfür. Alternativ kann anstelle des Pumpknopfs 104 auch ein Langloch vorgesehen sein, das die Betätigung der Pumpe 22 durch direkten Zugriff ermöglicht.

Denkbar wäre alternativ und weiterhin auch eine Betätigung des Pumpvorgangs durch ein geeignetes, beispielsweise mehrfaches oder mehrstufiges Verdrehen des Basismoduls 80 gegen das Reservoirmodul 82, sofern die Linsenkammern 16 währenddessen verschlossen bleiben. Die Herbeiführung einer Pumpwirkung auf diese Weise (z.B 6-mal drehen) würde einen zusätzlichen Pumpknopf dann überflüssig machen. Eine Drehung könnte beispielsweise 0,5 ml Flüssigkeit in die Linsenkammern befördern (2 x 0,25 ml). Die Dosierung wäre somit sehr präzise.

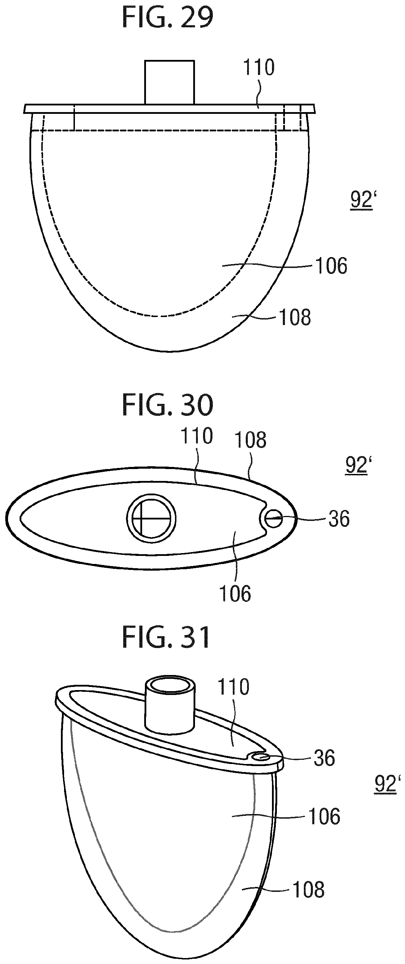

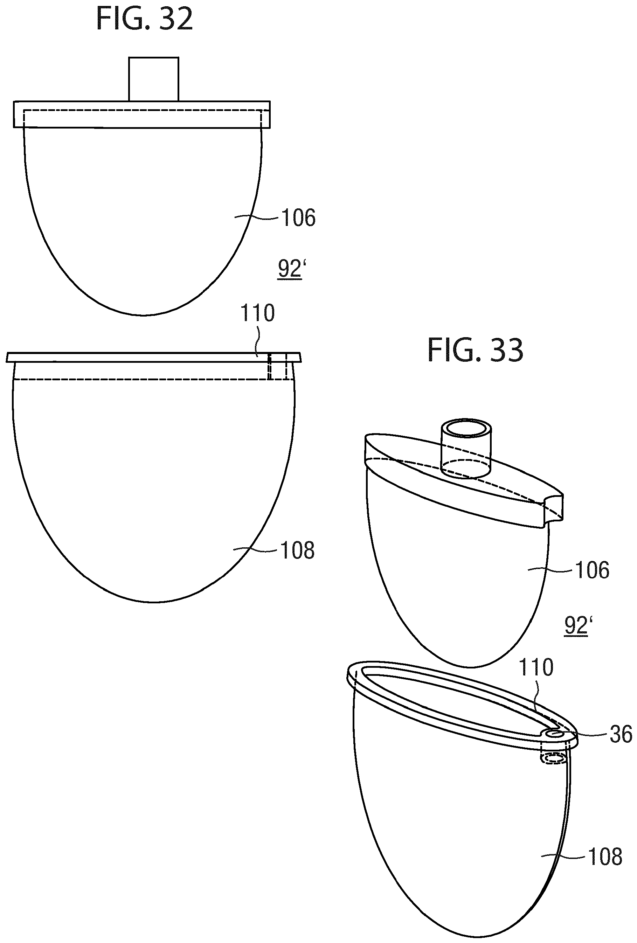

Eine alternative, als eigenständig erfinderisch angesehene Ausführungsform des Reservoirbeutels 92' ist in den Figs. 29 - 33 gezeigt. Wie diesen Darstellungen entnehmbar ist, ist der Reservoirbeutel 92' in dieser Ausführungsform mehrteilig ausgeführt, wobei ein innerer Reservoirbeutel 106 das eigentliche Reservoir bildet und zur Vorhaltung der Linsenflüssigkeit vorgesehen ist. Der innere Reservoirbeutel 106 ist dabei in montiertem Zustand innerhalb eines äußeren Reservoirbeutels 108 angeordnet. Dieser ist ursprünglich leer, also nicht befüllt, und dient als Auffangbehälter für die verbrauchte Linsenflüssigkeit nach dem Spülvorgang. Dazu ist das Kanalsystem 34 derart ausgelegt, dass die Auslassöffnung 36 für die verbrauchte Linsenflüssigkeit nicht am Außengehäuse 6 platziert ist, sondern in der Deckelplatte 110 des Reservoirbeutels 92' derart angeordnet ist, dass sie in den von den Reservoirbeuteln 106, 108 gebildeten Zwischenraum mündet. Damit ist kein externer Auslass mehr notwendig; der gesamte Reinigungsvorgang für die Linsen läuft im Inneren des Aufbewahrungs- und Reinigungsbehälters ab.

### Bezugszeichenliste

- 1, 1': Kontaktlinsen-Aufbewahrungs- und Reinigungsbehälter
- 2: Kartusche
- 4: Reservoir
- 6: Außengehäuse
- 8: Boden
- 10: Scharnier
- 12: Gehäusedeckel
- 14: Deckelverschluss
- 16: Kontaktlinsenkammer
- 18: Kammerdeckel
- 20: Kammerboden
- 22: Pumpvorrichtung
- 24: Dichtlippe
- 26: Sichtfenster
- 28: Anzeigeelement
- 30: Obere Kartuschenschale
- 32: Untere Kartuschenschale
- 34: Kanalsystem
- 36: Auslassöffnung
- 38: Kanalstück
- 40: Kanalplatte
- 42, 44: Rückschlagventil
- 46: Zuströmkanal
- 48: Einströmloch
- 50: Ausströmloch
- 52: Abströmkanal
- 54: Trägersteg
- 56: Befüllöffnung
- 58: Verschlusstopfen
- 60: Energiespeicher
- 62: Akkumulator

- 64: Ladebuchse
- 66: Schlauchpumpe
- 70: Bedienteil
- 80: Basismodul
- 82: Reservoirmodul
- 84: Verbindungstecker
- 86: Frontplatte
- 88: Gehäuseschale
- 90: Innenvolumen
- 92,92': Reservoirbeutel
- 94: Formteil
- 96: Verbindungsflansch
- 98, 100: Stirnfläche
- 101: Scharnier
- 102: Doppelpfeil
- 104: Pumpknopf
- 106: innerer Reservoirbeutel
- 108: äußerer Reservoirbeutel
- 110: Deckelplatte

## Patentansprüche

1. Kontaktlinsen-Aufbewahrungs- und Reinigungsbehälter (1) mit einer Anzahl von verschließbaren Kontaktlinsenkammern (16), denen mittels einer als Membranpumpe ausgestalteten Pumpvorrichtung (22) über ein Kanalsystem (34) Kontaktlinsenflüssigkeit aus einem Flüssigkeitsreservoir (4) zuführbar ist, wobei über das Kanalsystem (34) das Flüssigkeitsreservoir (4) flüssigkeitsseitig über die Kontaktlinsenkammer (16) oder die Kontaktlinsenkammern (16) mit einer Auslassöffnung (36) verbindbar ist, wobei das Flüssigkeitsreservoir (4) in eine in einem Außengehäuse (6) angeordnete Kartusche (2) integriert ist.

2. Behälter (1) nach Anspruch 1, bei dem das Kanalsystem (34) in einem separaten Gehäuseteil integriert ist.

3. Behälter (1) nach Anspruch 1 oder 2, bei dem die oder jede Kontaktlinsenkammer (16) jeweils durch einen mit einem zugeordneten Kammerdeckel (18) verschließbaren Kammerboden (20) gebildet ist, wobei der Kammerboden (20) durch eine Vertiefung in der Oberfläche der Kartusche (2) gebildet ist, und wobei der jeweilige Kammerdeckel (18) am Außengehäuse (6) angeordnet ist.

4. Behälter (1) nach Anspruch 3, bei dem der oder jeder Kammerdeckel (18) als verformbare Membran ausgeführt ist, die mit einem von außerhalb des Außengehäuses (6) einsehbaren Anzeigeelement (28) zusammenwirkt.

5. Behälter (1) nach einem der Ansprüche 1 bis 4, dessen Flüssigkeitsreservoir (4) wiederbefüllbar ist.

6. Behälter (1) nach einem der Ansprüche 1 bis 5, umfassend einen Energiespeicher (60) für elektrische Energie.

7. Behälter (1) nach Anspruch 6 in Verbindung mit Anspruch 3, an dessen Energiespeicher (60) eine Anzahl von dem oder den Kammerdeckeln (18) zugeordneten Vibrationselementen angeschlossen ist.

8. Behälter (1) nach Anspruch 6 oder 7, an dessen Energiespeicher (60) eine elektrische Anzeigeeinheit angeschlossen ist.

9. Behälter (1) nach einem der Ansprüche 6 bis 8, an dessen Energiespeicher (60) ein Aktivierungsmodul zur Anregung von elektroluminiszierenden Eigenschaften der Kontaktlinsen angeschlossen ist.

## Claims

1. A contact lens storage and cleansing container (1) comprising a number of closable contact lens chambers (16), to which contact lens fluid can be fed from a fluid reservoir (4) by means of a pump device (22) in the form of a diaphragm pump via a channel system (34), wherein the fluid reservoir (4) can be connected, at the fluid end, by the channel system (34) to an outlet opening (36) via the contact lens chamber (16) or the contact lens chambers (16), and wherein the fluid reservoir (4) is integrated into a cartridge (2) arranged in an external housing (6).

2. The container (1) according to claim 1, wherein the channel system (34) is integrated into a separate housing part.

3. The container (1) according to claim 1 or 2, wherein the or each contact lens chamber (16) is respectively formed by a chamber bottom (20) that can be closed with an assigned chamber cover (18), wherein the chamber bottom (20) is formed by a depression in the surface of the cartridge (2), and wherein the respective chamber cover (18) is arranged on the external housing (6).

4. The container (1) according to claim 3, wherein the or each chamber cover (18) is designed in the form of a deformable diaphragm, which interacts with a display element (28) that is visible from outside the external housing (6).

5. The container (1) according to one of claims 1 to 4, wherein the fluid reservoir (4) of the container is refillable.

6. The container (1) according to one of claims 1 to 5, comprising an energy storage (60) for electric energy.

7. The container (1) according to claim 6 in conjunction with claim 3, wherein a number of vibration elements, which are assigned to the chamber cover or the chamber covers (18), is connected to the energy storage (60) of the container.

8. The container (1) according to claim 6 or 7, wherein an electric display unit is connected to the energy storage (60) of the container.

9. The container (1) according to one of claims 6 to 8, wherein an activation module for exciting electroluminescent properties of the contact lenses is connected to the energy storage (60) of the container.

## Revendications

1. Récipient de rangement et de nettoyage (1) de lentilles de contact, doté d'un nombre de compartiments (16) pour lentilles de contact verrouillables, auxquels à l'aide d'un dispositif de pompe (22), conçu sous la forme d'une pompe à membrane, par l'intermédiaire d'un système de canalisation (34), du liquide pour lentilles de contact peut être alimenté à partir d'un réservoir à liquide (4), par l'intermédiaire du système de canalisation (34), le réservoir à liquide (4) étant susceptible d'être relié du côté liquide par l'intermédiaire du compartiment (16) pour lentille de contact ou des compartiments (16) pour lentilles de contact avec un orifice de sortie (36), le réservoir à liquide (4) étant intégré dans une cartouche (2) placée dans un boîtier externe (6).

2. Récipient (1) selon la revendication 1, sur lequel le système de canalisation (34) est intégré dans une partie séparée du boîtier.

3. Récipient (1) selon la revendication 1 ou 2, sur lequel le ou chaque compartiment (16) pour lentille de contact est formé par chaque fois un fond de compartiment (20) verrouillable avec un couvercle de compartiment (18) associé, le fond de compartiment (20) étant formé par un creux dans la surface de la cartouche (2) et le couvercle de compartiment (18) respectif étant placé sur le boîtier externe (6).

4. Récipient (1) selon la revendication 3, sur lequel le ou chaque couvercle de compartiment (18) est réalisé sous la forme d'une membrane déformable, qui coopère avec un élément d'affichage (28) visible à partir de l'extérieur du boîtier externe (6).

5. Récipient (1) selon l'une quelconque des revendications 1 à 4, dont le réservoir à liquide (4) est rechargeable.

6. Récipient (1) selon l'une quelconque des revendications 1 à 5, comprenant un accumulateur d'énergie (60) pour de l'énergie électrique.

7. Récipient (1) selon la revendication 6, en association avec la revendication 3, sur l'accumulateur d'énergie (60) duquel est raccordé un nombre d'éléments vibratoires, associés au ou aux couvercle(s) de compartiment (18).

8. Récipient (1) selon la revendication 6 ou 7, sur l'accumulateur d'énergie (60) duquel est raccordée une unité d'affichage électrique.

9. Récipient (1) selon l'une quelconque des revendications 6 à 8, sur l'accumulateur d'énergie (60) duquel est raccordé un module d'activation, destiné à stimuler des propriétés électroluminescentes des lentilles de contact.
